# EUROPEAN PATENT APPLICATION

(11) **EP 3 884 912 A1**
(43) Date of publication of application: **29.09.2021**
(21) Application number: 20165771.5
(22) Date of filing: 26.03.2020
(51) Int. Cl.: A61F 2/966, A61M 39/10, A61M 25/00

(54) **STENT DELIVERY SYSTEM**

(71) Applicant: B. Braun Melsungen AG, 34212 Melsungen (DE)
(72) Inventor: Murphy, Brian, Bray, Co. Wicklow, A 98 Y6WO (IE)
(74) Representative: Patentanwälte Ruff, Wilhelm, Beier, Dauster & Partner mbB

(57) **Abstract**

The invention relates to a stent delivery system comprising: a handling device having a housing and an actuator element mounted on the housing to be movable; a catheter arrangement having an inner shaft which has a first lumen and a proximal end fixed at least indirectly on the housing, and having an outer sheath disposed coaxially to the inner shaft, with an inner shell surface of the outer sheath spaced radially from an outer shell surface of the inner shaft, thereby forming an annular second lumen, and having at least one stent which is disposed within the annular second lumen; wherein the actuator element is in operative connection to the outer sheath such that, for release of the at least one stent, the outer sheath is displaceable relative to the inner shaft in the proximal direction by actuating the actuator element; and including a Luer port disposed proximally on the housing, with a fluid inlet of the Luer port being connected in fluid communication to at least the first lumen; wherein a fluid conduit assembly is provided and associated with the Luer port, the fluid inlet of the Luer port being connected in fluid communication to the first lumen and to the second lumen by means of the fluid conduit assembly, whereby a fluid flowing into the fluid inlet is led simultaneously into the first lumen and into the second lumen.

## Description

The invention relates to a stent delivery system.

It is well known to employ intravascular endoprostheses delivered percutaneously for the treatment of diseases of various body vessels. Such intravascular endoprostheses are commonly referred to as "stents". A stent is a generally longitudinal tubular device of biocompatible material having holes or slots that define a flexible framework that allows radial expansion of the stent, by a balloon catheter or the like, or alternatively by self-expansion due to shape memory characteristics of the material within the body vessel. The flexible framework is configured to allow the stent to be compressed into a smaller outer diameter so that it can be mounted inside a stent delivery system.

The stent delivery system is used to convey the stent to a desired location within the body vessel, and to release the stent in position. Upon release the stent may self-expand into a larger outer diameter.

WO 2019/053508 A1 discloses a stent delivery system having a handling device and a catheter arrangement. The catheter arrangement comprises an inner shaft which has a first lumen and a proximal end being fixed at least indirectly on a housing of the handling device. The catheter arrangement further comprises an outer sheath disposed coaxially to the inner shaft, with an inner shell surface of the outer sheath spaced radially from an outer shell surface of the inner shaft, thereby forming an annular second lumen. The catheter arrangement further comprises a stent which is disposed within the annular second lumen. The handling device comprises an actuator element that is in operative connection to the outer sheath such that, for release of the stent, the outer sheath is displaceable relative to the inner shaft in the proximal direction by actuating the actuator element. The stent delivery system includes a Luer port disposed proximally on the housing, with a fluid inlet of the Luer port being connected in fluid communication to the first lumen. The known stent delivery system includes an additional Luer port disposed distally on the housing, with a fluid inlet of the additional Luer port being connected in fluid communication to the annular second lumen. In that context, the first lumen can be flushed by passing a fluid into the proximal Luer port, and the annular second lumen can be flushed by passing a fluid through the additional Luer port. As a result, the flushing out of the first lumen and the annular second lumen requires two separate flushing procedures.

### SUMMARY OF THE INVENTION

It is the object of the present invention to provide a stent delivery system that allows a simplified handling during the flushing of the catheter arrangement.

According to one aspect, a stent delivery system is provided comprising: a handling device having a housing and an actuator element mounted on the housing to be movable; a catheter arrangement having an inner shaft which has a first lumen and a proximal end fixed at least indirectly on the housing, and having an outer sheath disposed coaxially to the inner shaft, with an inner shell surface of the outer sheath spaced radially from an outer shell surface of the inner shaft, thereby forming an annular second lumen, and having at least one stent which is disposed within the annular second lumen; wherein the actuator element is in operative connection to the outer sheath such that, for release of the at least one stent, the outer sheath is displaceable relative to the inner shaft in the proximal direction by actuating the actuator element; and including a Luer port disposed proximally on the housing, with a fluid inlet of the Luer port being connected in fluid communication to at least the first lumen; wherein a fluid conduit assembly is provided and associated with the Luer port, the fluid inlet of the Luer port being connected in fluid communication to the first lumen and to the second lumen by means of the fluid conduit assembly, whereby a fluid flowing into the fluid inlet is led simultaneously into the first lumen and into the second lumen. Owing to the solution according to the invention, significantly simplified flushing out of the catheter arrangement can be achieved, as compared to the prior art. Namely, instead of two separate Luer ports, merely the one proximal Luer port is provided and connected in fluid communication to both the first lumen and the second lumen by means of the fluid conduit assembly. Thereby, the first lumen and the second lumen can be flushed out together during a single flushing procedure and, thus, simultaneously.

In one embodiment, the fluid conduit assembly includes an elongate tubular member which is disposed coaxially to the inner shaft and the outer sheath and radially between the inner shaft and the outer sheath, wherein an annular third lumen is formed between an inner shell surface of the tubular member and the outer shell surface of the inner shaft, by means of which third lumen the annular second lumen is connected in fluid communication to the fluid inlet of the Luer port. In simple terms, the elongate tubular member acts as a transition between the Luer port and the annular second lumen. Thereby, a fluid flowing into the fluid inlet of the Luer port is can be lead into the annular second lumen via the annular third lumen. The elongate tubular member can in particular be configured as a tube, hose, cannula or the like. Preferably, the elongate tubular member is composed of a dimensionally stable material. As a result, unintentional deformation of the elongate tubular member and along with it impairment of the annular third lumen can be counteracted.

In one embodiment, a proximal end of the tubular member is fixedly inserted into a distal end of the Luer port, and a proximal end of the outer sheath is pushed onto a distal end of the tubular member and movable axially. This embodiment of the invention allows a particularly simple construction. For that purpose, the proximal end of the tubular member interacts directly with the distal end of the Luer port and the proximal end of the outer sheath interacts directly with the distal end of the tubular member. Preferably, the distal end of the Luer port has a through hole into which the proximal end of the tubular member is inserted. For that purpose, the proximal end of the tubular member can be joined into the through hole, in a fluid-tight manner, for example by adhesive bonding, welding, screwing or any other way known to a person skilled in the art. In each case, in cooperation with the inner shaft, the annular third lumen is formed thereby. On its distal end, the tubular member cooperates with the outer sheath. For releasing the stent, the outer sheath is displaceable in the proximal direction relative to the inner shaft in a generally well-known manner. In order to ensure this displaceablility of the outer sheath, the outer sheath with its proximal end is pushed onto the distal end of the tubular member in axial mobility. In other words, the distal end of the tubular member is inserted into the proximal end of the outer sheath. Preferably, a diameter of an outer shell surface of the tubular member and a diameter of the inner shell surface of the outer sheath are matched to each other such that a fluid-tight sealing of a sealing gap between the inner shell surface and the outer shell surface and, at the same time, smooth axial mobility of the outer sheath are ensured.

In one embodiment, a sealing element is provided, by means of which a sealing gap between the inner shell surface of the outer sheath and an outer shell surface of the tubular member is sealed in a fluid-tight manner. As a result, an unintentional leaking of fluid through the sealing gap can be counteracted. The sealing element is preferably fixedly connected to the proximal end of the outer sheath and, thus, during releasing of the stent, is displaceable in the proximal direction together with the outer sheath relative to the inner shaft and also to the tubular member. Preferably, the sealing element has an annular shape and engages around the outer shell surface of the tubular member in a fluid-tight manner.

In one embodiment, the tubular member is a cannula made of metal. As a result, a particularly simple construction and cost-efficient manufacture are achieved.

In one embodiment, the fluid conduit assembly includes an adapter element, by means of which the proximal end of the inner shaft is fixed to the Luer port. Accordingly, the adapter element is used, in particular for fixing the proximal end of the inner shaft to the Luer port. If the fluid conduit assembly according to any of the preceding embodiments includes a tubular element, the adapter element is preferably configured such that a coaxial orientation of the inner shaft in relation to the tubular element is ensured.

In one embodiment, the adapter element is inserted into a female Luer cone of the Luer port and includes a first fluid passage connecting the fluid inlet to the first lumen in fluid communication and at least one second fluid passage connecting the fluid inlet to the second lumen in fluid communication. Accordingly, in this the embodiment the adapter element is additionally used for a transfer of the fluid between the fluid inlet of the Luer port and the first lumen and a transfer between the fluid inlet and the second lumen. For that purpose, the adapter element includes the first fluid passage and the second fluid passage. By means of the first fluid passage, the fluid inlet and the first lumen are connected in fluid communication. By means of the second fluid passage, the fluid inlet and the second lumen are connected in fluid communication, at least indirectly. If the fluid conduit assembly according to any of the preceding embodiments includes a tubular element which provides a annular third lumen by interaction with the inner shaft, the second lumen is connected in fluid communication to the fluid inlet via the third lumen and from there further via the second fluid passage. Starting from the fluid inlet, the adapter element provides a type of fluidic branching, and a fluid stream flowing into the fluid inlet branches off, on the one hand, in the direction towards the first lumen and, on the other hand, in the direction towards the second lumen.

In one embodiment, the first fluid passage extends coaxially through the adapter element and includes a proximal end, into which the fluid inlet of the Luer port leads, and a distal end, into which the proximal end of the inner shaft is fixedly inserted. A particularly simple configuration is permitted thereby.

In one embodiment, the at least one second fluid passage is provided by a radial recess of a conical outer contour of the adapter element, wherein the conical outer contour is inserted into the female Luer cone of the Luer port. The conical outer contour of the adapter element is inserted into the female Luer cone of the Luer port, thereby forming a conical seat connection. The conical outer contour is similar to a male Luer cone. The configuration of such a male Luer cone is well-known per se. However, the conical outer contour of the adapter element - in contrast to a conventional male Luer cone - does not provide a fluid-tight conical seat connection. This is due to the at least one radial recess of the conical outer contour which form the at least one second fluid passage. Owing to the radial recess, a fluid led into the fluid inlet can pass between the conical outer contour and the female Luer cone in the direction towards the second lumen. Owing to this embodiment of the invention, a space-efficient arrangement of the second passage, thus a compact configuration of the adapter element and, finally, a particularly advantageous configuration of the fluid conduit assembly is achieved.

In one embodiment, the adapter element has a plurality of radial recesses disposed mutually adjacent in the circumferential direction, and thereby a plurality of second fluid passages disposed mutually adjacent in the circumferential direction. Owing to this embodiment of the invention, a particularly low-resistance branching of the fluid flow in the region of the adapter element can be achieved. Namely, the plurality of second fluid passages disposed mutually adjacent in the circumferential direction form a type of parallel circuit which exhibits a lower flow resistance as compared to a single second fluid passage. In this embodiment of the invention, the adapter element has a star-shaped cross section due to the plurality of radial recesses disposed mutually adjacent in the circumferential direction.

### BRIEF DESCRIPTION OF THE DRAWINGS

In the following, an embodiment of the invention will be described in detail with reference to the drawings. Throughout the drawings, the same elements will be denoted by the same reference numerals. The drawings schematically show:
- Fig. 1: in an isometric view an embodiment of a stent delivery system according to the invention, including a handling device and a catheter arrangement with a plurality of stents, wherein the catheter arrangement is illustrated with partial sections;
- Fig. 2: the stent delivery system according to Fig. 1 in the region of the handling device in a further isometric view;

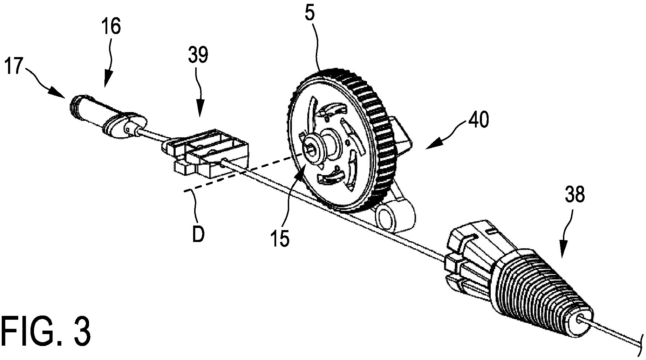

- Fig. 3: the stent delivery system according to Figs. 1 and 2 in a view corresponding to Fig. 2 with individual components and/or sections of the handling device hidden in the drawing;
- Fig. 4: an enlarged longitudinal sectional view of the stent delivery system in the region of a proximal Luer port which is associated with a fluid conduit assembly for connection to different lumina of the catheter arrangement in fluid communication;
- Fig. 5: an enlarged detail illustration of a region V according to Fig. 4;
- Fig. 6: a greatly simplified schematic longitudinal sectional view of the fluid conduit assembly;
- Fig. 7: in an isometric view an adapter element of the fluid conduit assembly;
- Fig. 8: the adapter element according to Fig. 7 in an isometric longitudinal sectional view; and
- Fig. 9: the adapter element according to Figs. 7 and 8 in a longitudinal sectional view in a radial direction of viewing.

### DETAILED DESCRIPTION OF EMBODIMENTS OF THE INVENTION

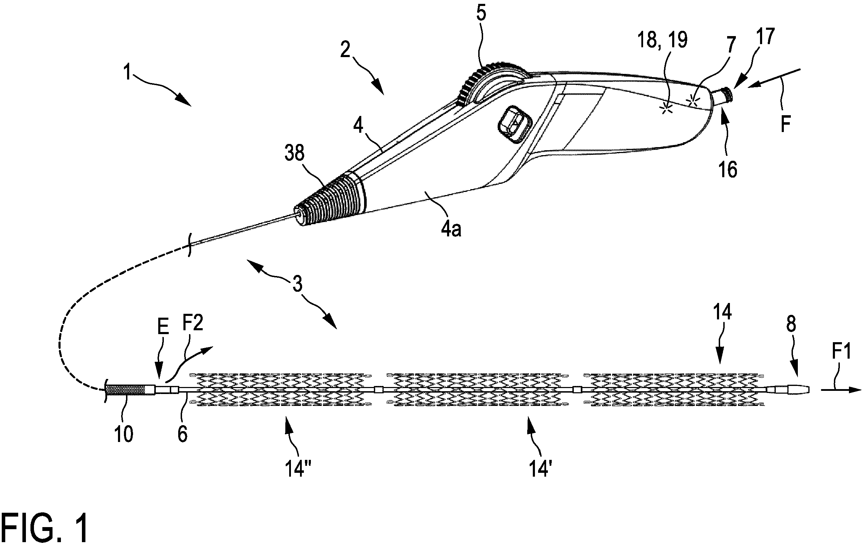

According to Fig. 1, a stent delivery system 1 comprises a handling device 2 and a catheter arrangement 3. The stent delivery system 1 is intended for use in treatment of stenosis in blood vessels.

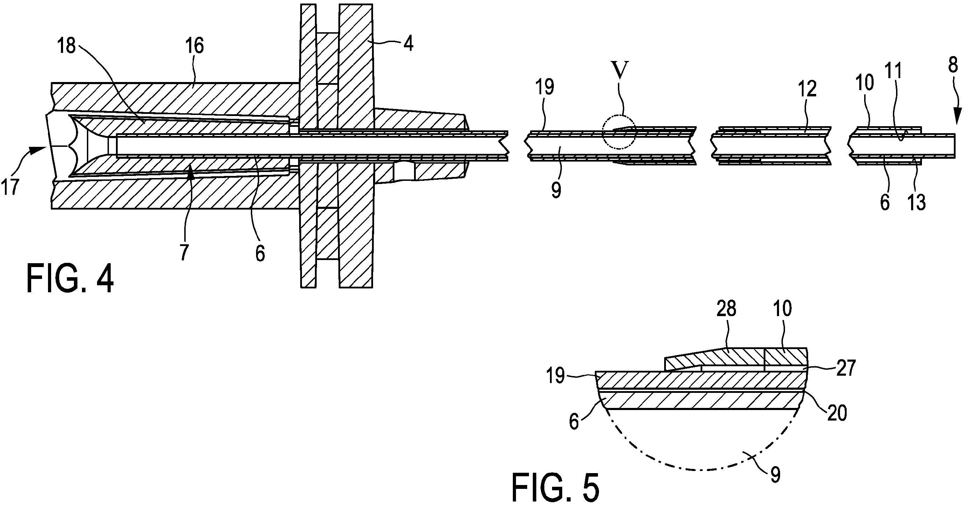

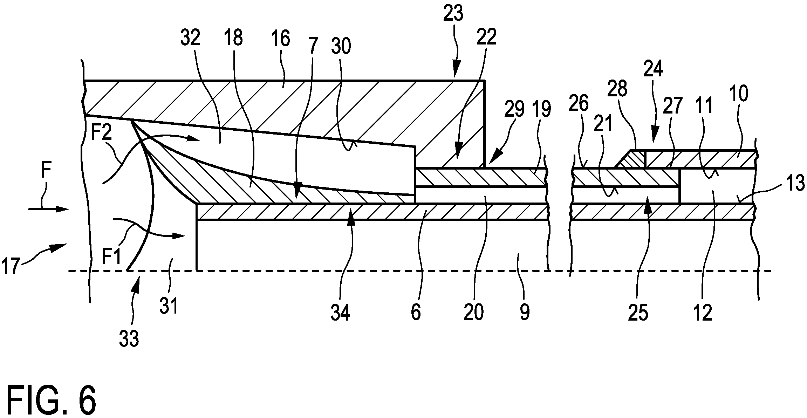

The handling device 2 has a housing 4 with an actuator element 5 mounted on the housing 4 to be movable. The catheter arrangement 3 has an inner shaft 6 with a proximal end 7, a distal end 8 and a first lumen 9 extending longitudinally between the proximal end 7 and the distal end 8. The proximal end 7 of the inner shaft 6 is at least indirectly fixed to the housing 4 in a manner that will be described in more detail below (Figs. 4, 6). Further, the catheter arrangement 3 has an outer sheath 10 disposed coaxially to the inner shaft 6, with an inner shell surface 11 of the outer sheath 10 spaced radially from an outer shell surface 13 of the inner shaft 6, thereby forming an annular second lumen 12 (Figs. 4, 6). Further, the catheter arrangement 3 includes a stent 14 which is received radially between the inner shaft 6 and the outer sheath 10 in a condition of the catheter arrangement 3 not illustrated in more detail in the drawing. The stent 14 is disposed within the annular second lumen 12. The actuator element 5 is in operative connection to the outer sheath 10 in a manner generally known to a person skilled in the art such that, for release of the stent 14, the outer sheath 10 is displaceable relative to the inner shaft 6 in the proximal direction by actuating the actuator element 5. The above described release of the stent 14 takes place starting from a displacement position of the outer sheath 10 not illustrated in more detail in the drawing, wherein a distal end E of the outer sheath 10 is essentially flush with the distal end 8 of the inner shaft 6. In this condition, the stent 14 is compressed in the radial direction between the outer shell surface 11 and the outer shell surface 13. Following the above mentioned retraction of the outer sheath 10 in the proximal direction, the stent 14 is released in the radial direction, and thereby may expand in a generally well-known manner. With reference to Fig. 1, a corresponding released and expanded condition of the stent 14 is illustrated.

In the embodiment shown, the actuator element 5 is a thumbwheel mounted in the housing 4 to be rotatable about a rotation axis D. The thumbwheel is in operative connection to the outer sheath 10 via a flexible pull member not illustrated in more detail in the drawing. For that purpose, the flexible pull member is held, with one end, on a winding spool 15 to be windable, which winding spool 15 is connected to the thumbwheel in a torque-transmitting manner and rotating together with the latter about the rotation axis D. With the other end, the flexible pull member engages on a proximal region of the outer sheath 10 for transfer of pulling force. However, such a configuration is not mandatory. In an embodiment not shown in the drawing, the actuator element may be, for example, a pusher, lever or the like capable of linear movement, which is in operative connection to the outer sheath 10 for displacement of the latter using a pull member or any other ways and means known to a person skilled in the art.

Moreover, the stent delivery system 1 includes a Luer port 16 with a fluid inlet 17 disposed proximally on the housing 4. The Luer port 16 is provided for connection in fluid communication to a complementary Luer port of a fluid-supplying component in a generally well-known manner.

The Luer port 16 is associated with a fluid conduit assembly 18, 19 by means of which the fluid inlet 17 is connected in fluid communication to the first lumen 9 and to the second lumen 12, whereby a fluid flowing into the fluid inlet 17 is led simultaneously into the first lumen 9 and into the second lumen 12.

Before discussing the further specific configuration of the handling device 2 and the catheter arrangement 3, the further features in the context of the fluid conduit assembly 18, 19 are first explained, in particular with reference to Figs. 4 to 9:
In the present case, the fluid conduit assembly 18, 19 includes an elongate tubular member 19 and an adapter element 18.

The elongate tubular member 19 extends in the longitudinal direction coaxially to the inner shaft 6 and the outer sheath 10. Moreover, the elongate tubular member 19 is disposed radially between the inner shaft 6 and the outer sheath 10. As a result, an annular third lumen 20 is formed between an inner shell surface 21 of the tubular member 19 and the outer shell surface 13 of the inner shaft 6. By means of the annular third lumen 20, the second lumen 12 is connected in fluid communication to the fluid inlet 17 (Fig. 6).

As is further apparent with reference to Figs. 4 and 6, a proximal end 22 of the elongate tubular member 19 is fixedly inserted in a distal end 23 of the Luer port 16. Moreover, a proximal end 24 of the outer sheath 10 is pushed onto a distal end 25 of the tubular member 19 and movable axially. This is to ensure that the outer sheath 10 is displaceable for release of the stent 14 in the above mentioned manner by rotating actuation of the thumbwheel 5 in the proximal direction. Thus, this displacement move occurs both relative to the inner shaft 6 and to the elongate tubular member 19

In the embodiment shown, the proximal end 22 is fixedly inserted in a through hole 29 provided on the distal end 23. The through hole 29 extends coaxially to the elongate tubular member 19. The through hole 29 provides a fluid outlet of the Luer port 16. In the embodiment shown, the proximal end 22 is adhesively bonded in the distal end 23 and the through hole 29, respectively, however, this is not mandatory.

Furthermore, a sealing gap 27 is formed between the inner shell surface 11 of the outer sheath 10 and an outer shell surface 26 of the elongate tubular member 19. In order to counteract unintended leaking of fluid through the sealing gap 27 during flushing the second lumen 12, the sealing gap 27 is sealed in a fluid-tight manner. In the embodiment shown, a sealing element 28 is provided for that purpose. The sealing element 28 is fixed to the proximal end 24 in a manner known to a person skilled in the art and movable together with the outer sheath 10.

In an embodiment not shown, the fluid-tight sealing of the sealing gap 27 is achieved in that the diameter of the outer shell surface 26 and the diameter of the inner shell surface 11 are matched accordingly. In that context, the sealing element 28 does not need to be present in each embodiment.

In the embodiment shown, the adapter element 18 is used for fixing of the proximal end 7 of the inner shaft 6 at least indirectly on the housing 4 (Figs. 4, 6). In that context, the proximal end 7 is fixed to the Luer port 16 by means of the adapter element 18 in a manner that will be described in more detail below. Also, the adapter element 18 is used for fluidic branching, in a manner that will be described in more detail below, by means of which branching a fluid stream F led into the fluid inlet 17 is branched in the direction of the first lumen 9 and in the direction of the second lumen 12.

In the embodiment shown the adapter element 18 is inserted in a female Luer cone 30 of the Luer port 16 and includes a first fluid passage 31 and at least a second fluid passage 32.

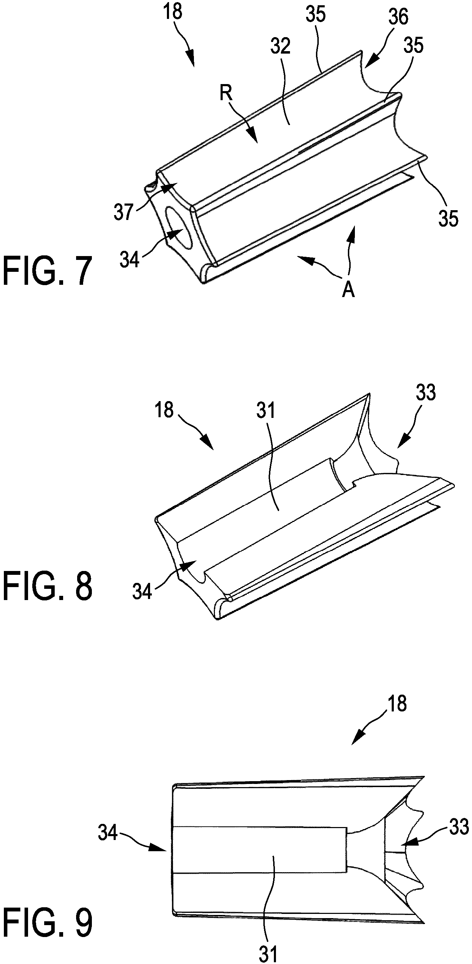

The first fluid passage 31 extends coaxially through the adapter element 18 and is oriented coaxially to the proximal end 7 of the inner shaft 6. The first fluid passage 31 has a proximal end 33 and a distal end 34 (Figs. 8, 9). As is apparent in particular with reference to Fig. 6, the fluid inlet 17 leads into the proximal end 33 of the first fluid passage 31. In contrast, the proximal end 7 of the inner shaft 6 is fixedly inserted in the distal end 34 of the first fluid passage 31. In the vicinity of the distal end 34, the first fluid passage 31 is configured in the shape of a circular cylindrical hole matched to the diameter of the outer shell surface 13. Accordingly, a fluid-tight connection between the outer shell surface 13 of the inner shaft 6 and the hole is provided. In the vicinity of the proximal end 33, the first fluid passage 31 has a funnel-shaped configuration which tapers starting from the fluid inlet 17 in the distal direction. As a result, the first lumen 9 is connected via the first fluid passage 31 in fluid communication to the fluid inlet 17.

As is illustrated in particular with reference to Fig. 7, in the present case, the adapter element 18 has a plurality of second fluid passages 32 disposed mutually adjacent in the circumferential direction, which fluid passages are identical in configuration and design. For reasons of brevity, merely one of the second fluid passages 32 is discussed in detail. The explanations given thereto apply correspondingly in view of the further second fluid passages 32.

The second fluid passage 32 is provided by a radial recess R of a conical outer contour A of the adapter element 18 (Fig. 7). For fixation of the adapter element 18, the conical outer contour A cooperates with the female Luer cone 30 of the Luer port 16 (Fig. 6). In other words, the conical outer contour A can be described as an imaginary envelope surface which encloses the shape of the adapter element 18, said shape is evidently not conical per se, on the outer circumference thereof. In the embodiment shown, the conical outer contour A spans in the circumferential direction of the adapter element 18 mutually spaced web sections 35 (Fig. 7), between which web 35 sections the radial recess R is disposed and forming the second fluid passage 32. The web sections 35 extend axially between a proximal and a distal end of the adapter element 18. The same applies to the radial recess R. Correspondingly, the second fluid passage 32 has a proximal end 36 and a distal end 37 (Fig. 7).

Since in the embodiment shown, a plurality of radial recesses R are disposed mutually adjacent in the circumferential direction, the adapter element 18 has a star shape in cross section.

With reference to Fig. 6 it is apparent that the conical outer contour A - in contrast to a conventional male Luer cone - does not cooperate with the female Luer cone 30 forming a fluid-tight connection. Rather, the radial recesses R allows that the fluid stream F led in through the fluid inlet 17 flows at least partially along an outer side of the adapater element, namely through the second fluid passage 32.

For flushing the catheter arrangement 3, the fluid stream F is led into the fluid inlet 17 of the Luer port 16. For that purpose, the Luer port 16 can be connected to a medical hose line, a syringe, a liquid container or the like, in a manner known to a person skilled in the art. By means of the fluid conduit assembly 18, 19, the fluid stream F is led starting from the fluid inlet 17 partially in the direction of the first lumen 9 and partially in the direction of the annular second lumen 12. In other words the fluid stream F branches off into a first fluid partial stream F1 and a second fluid partial stream F2. This feature is schematically indicated by corresponding arrows in Fig. 6.

The first fluid partial stream F1 enters the first fluid passage 31 via the proximal end 33 and from there in the proximal end 7 of the inner shaft 6 and, thus, in the first lumen 9. Thereby, the first fluid partial stream F1 is led in the distal direction through the first lumen 9 and out of distal end 8 thereof.

The second fluid partial stream F2, starting from the proximal end 36 of the second fluid passage 31 and the radial recess R, respectively, reaches in the distal direction up to the distal end 37. From there, the second fluid partial stream F2 enters the annular third lumen 20 via the proximal end 22 of the elongate tubular member 19, flushes the lumen 20 in the distal direction and enters the annular second lumen 12 via the distal end 25 of the elongate tubular member 19. The latter lumen 12 is thereby flushed in the distal direction and the second fluid partial stream F2 is discharged in the region of the distal end E of the outer sheath 10 (Fig. 1) from the annular second lumen 12. The distal end E is positioned in different locations relative to the inner shaft 6, depending on the displacement position of the outer sheath 10.

In the following, further detail of the configuration of the handling device 2 and the catheter arrangement 3 are discussed, however, are not to be interpreted as mandatory in view of the present invention.

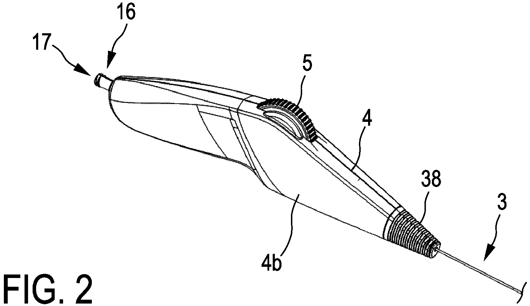

In the present case, the housing 4 has two housing halves 4a, 4b, namely a first housing half 4a (Fig. 1) and a second housing half 4b (Fig. 2). In the ready-for-use condition of the stent delivery system 1, shown with reference to the Figs. 1 and 2, the housing halves 4a, 4b are joined together in a generally well-known manner. In the present case, the housing 4 is manufactured from a dimensionally stable synthetic material. The housing 4 provides a handle which may be grasped by a hand such that the thumb of the respective hand is placed in the region of an upper side of the housing 4 for rotating actuation of the actuator element 5.

The Luer port 16 is configured as a female Luer port in a manner known to a person skilled in the art, and is fixed between the housing halves 4a, 4b in the joined condition of the housing 4.

Apart from that, in the embodiment shown, the catheter arrangement includes further stents, namely a second stent 14' and a third stent 14"which are disposed spaced from the stent 14 in the proximale direction and retained within the annular second lumen 12 in a corresponding manner. In a not shown embodiment, merely one stent is provided.

The catheter arrangement 3 extends in the region of the handling device 2 between a distal end and a proximal end of the housing 4 within the latter. The inner shaft 6 has a flexible design. The outer sheath 10 is manufactured from a braided material, in the embodiment shown, and connected in the region of its proximal end 24 to the flexible pull member, in a manner known to a person skilled in the art. The stent 14 is a self-expanding stent. The same applies to the further stents 14', 14".

Further, the handling device 2 includes a guiding tip 38 which is disposed on a distal end of the housing 4 and is held between the housing halves 4a, 4b. The catheter arrangement 3 extends, through the guiding tip 38 in the axial direction.

Furthermore, in the embodiment shown, the handling device 2 includes a deflecting and tensioning device 39 which is disposed in the axial direction of the catheter arrangement 3 between the actuator element 5 and the proximal end 7 of the inner shaft 6. The deflecting and tensioning device 39 is intended for deflecting and tensioning of said flexible pull member. Moreover, in the present case, a locking and unlocking mechanism 40 is provided which is intended for locking and unlocking the rotational mobility of the actuator element 5. Neither the deflecting and tensioning device 39 nor the locking and unlocking mechanism 40 are mandatory, therefore, a more detailed description thereof is omitted for reasons of brevity.

## Claims

1. Stent delivery system (1) comprising:
- a handling device (2) having a housing (4) and an actuator element (5) mounted on the housing (4) to be movable;
- a catheter arrangement (3) having an inner shaft (6) which has a first lumen (9) and a proximal end (7) fixed at least indirectly on the housing (4), and having an outer sheath (10) disposed coaxially to the inner shaft (6), with an inner shell surface (11) of the outer sheath (10) spaced radially from an outer shell surface (13) of the inner shaft (6), thereby forming an annular second lumen (12), and having at least one stent (14) which is disposed within the annular second lumen (12);
- wherein the actuator element (5) is in operative connection to the outer sheath (10) such that, for release of the at least one stent (14), the outer sheath (10) is displaceable relative to the inner shaft (6) in the proximal direction by actuating the actuator element (5);
- and including a Luer port (16) disposed proximally on the housing (4), with a fluid inlet (17) of the Luer port (16) being connected in fluid communication to at least the first lumen (9);
- **characterized in that** a fluid conduit assembly (18, 19) is provided and associated with the Luer port (16), the fluid inlet (17) of the Luer port (16) being connected in fluid communication to the first lumen (9) and to the second lumen (12) by means of the fluid conduit assembly (18, 19), whereby a fluid flowing into the fluid inlet (17) is led simultaneously into the first lumen (9) and into the second lumen (12).

2. Stent delivery system (1) according to claim 1, **characterized in that** the fluid conduit assembly (18, 19) includes an elongate tubular member (19) which is disposed coaxially to the inner shaft (6) and to the outer sheath (10) and radially between the inner shaft (6) and the outer sheath (10), wherein an annular third lumen (20) is formed between an inner shell surface (21) of the tubular member (19) and the outer shell surface (13) of the inner shaft (6), by means of which third lumen (20) the annular second lumen (12) is connected in fluid communication to the fluid inlet (17) of the Luer port (16).

3. Stent delivery system (1) according to claim 2, **characterized in that** a proximal end (22) of the tubular member (19) is fixedly inserted into a distal end (23) of the Luer port (16), and **in that** a proximal end (24) of the outer sheath (10) is pushed onto a distal end (25) of the tubular member (19) and movable axially.

4. Stent delivery system (1) according to claim 2 or 3, **characterized in that** a sealing element (28) is provided, by means of which a sealing gap (27) between the inner shell surface (11) of the outer sheath (10) and an outer shell surface (26) of the tubular member (19) is sealed in a fluid-tight manner.

5. Stent delivery system (1) according to any of claims 2 to 4, **characterized in that** the tubular member (19) is a cannula made of metal.

6. Stent delivery system (1) according to any of the preceding claims, **characterized in that** the fluid conduit assembly (18, 19) includes an adapter element (18), by means of which the proximal end (7) of the inner shaft (6) is fixed to the Luer port (16).

7. Stent delivery system (1) according to claim 6, **characterized in that** the adapter element (18) is inserted into a female Luer cone (30) of the Luer port (16) and includes a first fluid passage (31) connecting the fluid inlet (17) to the first lumen (9) in fluid communication and at least one second fluid passage (32) connecting the fluid inlet (17) to the second lumen (12) in fluid communication.

8. Stent delivery system (1) according to claim 7, **characterized in that** the first fluid passage (31) extends coaxially through the adapter element (18) and includes a proximal end (33), into which the fluid inlet (17) of the Luer port (16) leads, and a distal end (34), into which the proximal end (7) of the inner shaft (6) is fixedly inserted.

9. Stent delivery system (1) according to claim 7 or 8, **characterized in that** the at least one second fluid passage (32) is provided by a radial recess (R) of a conical outer contour (A) of the adapter element (18), wherein the conical outer contour (A) is inserted into the female Luer cone (30) of the Luer port (16).

10. Stent delivery system (1) according to claim 9, **characterized in that** the adapter element (18) has a plurality of radial recesses (R) disposed mutually adjacent in the circumferential direction, and thereby a plurality of second fluid passages (32) disposed mutually adjacent in the circumferential direction.
